# NEW EUROPEAN PATENT SPECIFICATION

(11) **EP 0 822 172 B2**
(45) Date of publication and mention of the opposition decision: **06.10.2004**
(45) Mention of the grant of the patent: 28.11.2001
(21) Application number: 97900750.7
(22) Date of filing: 22.01.1997
(51) Int. Cl.: C07C 43/12, C07C 41/01

(54) **PROCESS FOR PREPARING FLUOROMETHYL 1,1,1,3,3,3-HEXAFLUOROISOPROPYL ETHER**
VERFAHREN ZUR HERSTELLUNG VON FLUOROMETHYL-(1,1,1,3,3,3)-HEXAFLUOROISOPROPYLETHER
PROCEDE DE PREPARATION DE L'ETHER DE FLUOROMETHYLE 1,1,1,3,3,3-HEXAFLUORO-ISOPROPYLIQUE

(30) Priority: 21.02.1996 JP 3333996
(43) Date of publication of application: 04.02.1998
(73) Proprietor: CENTRAL GLASS COMPANY, LIMITED, Ube-shi, Yamaguchi-ken, 755 (JP)
(72) Inventor: KAWAI, Toshikazu, c/o Central Glass Co., Ltd., Kawagoe-shi, Saitama 350-11 (JP); WATANABE, Mineo, c/o Central Glass Co., Ltd., Kawagoe-shi, Saitama 350-11 (JP)
(74) Representative: Manitz, Finsterwald & Partner
(86) International application number: PCT/JP1997/000129
(87) International publication number: WO 1997/030961

(56) References cited:
- WO-A-93/12057
- JP-A- 1 319 449
- JP-A- 56 501 806
- US-A- 4 250 334
- US-A- 4 469 898

## Description

The present invention relates to a method of producing fluoromethyl 1,1,1,3,3,3-hexafluoroisopropyl ether, which is widely used as pharmaceutical and agrichemical products or intermediates thereof.

Several methods of producing fluoromethyl 1,1,1,3,3,3-hexafluoroisopropyl ether are known. For example, according to one method a mixture of concentrated sulfuric acid, hydrogen fluoride, paraformaldehyde and 1,1,1,3,3,3-hexafluoroisopropyl alcohol (hereinafter referred to as "HFIPA") is heated, and then the generated gas is trapped (U.S. Patent 4,469,898). U.S. Patent 4,250,334 discloses a similar method wherein HFIPA is added to a mixture comprising a stoichiometric excess of paraformaldehyde and hydrogen fluoride, plus sufficient sulfuric acid to sequester most of the water produced by the reaction. The mixture is maintained at a temperature of at least 57°C, to cause vapor formation by boiling of the fluoromethyl 1,1,1,3,3,3-hexafluoroisopropyl ether formed. The vapor is then collected, condensed, and purified by destillation. Another method comprises the addition of trioxane to hydrogen fluoride, and then the addition of HFIPA thereto (JP-T-7-502037). Other methods are also known. However, the known methods either result in various kinds of polyethers being produced as by-products, or their yields are very low. These polyethers are separated from fluoromethyl 1,1,1,3,3,3-hexafluoroisopropyl ether by destillation, and then are discarded.

In addition, a method is also known in which chloromethyl 1,1,1,3,3,3-hexafluoroisopropyl ether is fluorinated by potassium fluoride at high temperature and high pressure. However, the reaction conditions are severe, and furthermore the yield is merely 60%.

The present inventors have examined a method of producing high yields of fluoromethyl 1,1,1,3,3,3-hexafluoroisopropyl ether from formaldehyde or a polymer thereof.

According to a first method of the present invention it is possible to produce fluoromethyl 1,1,1,3,3,3-hexafluoroisopropyl ether by bringing a polyether represented by the following general formula (1) into contact with a medium comprising hydrogen fluoride, an accelerant and optionally formaldehyde,

R¹O(CH₂O)ₙR² (1)

where R¹ and R² are independently C₁-C₁₀ alkyl or haloalkyl groups, where halogen is fluorine, chlorine or bromine, n is an integer of from 1 to 10, and at least one of R¹ and R² is the (CF₃)₂CH-group, said polyether being a reactant for producing fluoromethyl 1,1,1,3,3,3-hexafluoroisopropyl ether.

Furthermore, the present invention provides a second method of producing fluoromethyl 1,1,1,3,3,3-hexafluoroisopropyl ether by bringing a polyether represented by the following general formula (2) and 1,1,1,3,3,3-hexafluoroisopropyl alcohol into contact with a medium comprising hydrogen fluoride, an accelerant and optionally formaldehyde,

R¹O(CH₂O)ₙR² (2)

where R¹ and R² are independently hydrogen, C₁-C₁₀ alkyl or haloalkyl groups, where halogen is fluorine, chlorine or bromine, n is an integer of from 1 to 10, and both of R¹ and R² are not hydrogen at the same time, said polyether being a reactant for producing fluoromethyl 1,1,1,3,3,3-hexafluoroisopropyl ether.

Concrete examples of R¹ and R² are alkyl groups such as methyl, ethyl, n-propyl, i-propyl, n-butyl, sec-butyl, t-butyl, pentyl, hexyl, heptyl and octyl, and residues which are isomers thereof, although no specific examples are given.

Furthermore, examples of the haloalkyl groups, in which fluorine and/or chlorine has been substituted for at least one hydrogen of these alkyl groups, are fluoromethyl, difluoromethyl, trifluoromethyl, fluoroethyl, difluoroethyl, 1-fluoroisopropyl, 1,1-difluoroisopropyl, 1,1,1-trifluoroisopropyl, 1,1,1,2-tetrafluoroisopropyl, pentafluoroisopropyl, hexafluoroisopropyl, and the like.

In the second method it is preferable that at least one of R¹ and R² is the fluoromethyl group or the hexafluoroisopropyl group.

The polyoxymethylene group is represented by a straight chain in the general formulae (1) and (2). It is not necessary to particularly limit the unit number (n) of the oxymethylene group. However, if it is too large, its behavior will become substantially similar to that of formaldehyde polymer if the method of the present invention is used. Thus, it becomes necessary to increase the amount added of the later mentioned HFIPA. With this, the use of a polyether of the general formula (1) and formula (2) does not make sense very much.

Concrete examples of polyethers of the general formulae (1) and (2) which are preferably used in the method of the present invention are:

(CF₃)₂CHO(CH₂O)ₙCH(CF₃)₂

where n is an integer of from 1 to 10,

(CF₃)₂CHO(CH₂O)ₙCH₂F

where n is an integer of from 1 to 10, and

(CF₃)₂CHO(CH₂O)ₙCH₃

where n is an integer of from 1 to 10.
Particularly preferable ones are:

(CF₃)₂CHO(CH₂O)ₐCH(CF₃)₂

where a is an integer of from 1 to 7,

(CF₃)₂CHO(CH₂O)_{b}CH₂F

where b is an integer of from 1 to 6, and

(CF₃)₂CHO(CH₂O)_{c}CH₃

where c is an integer of from 1 to 4.
Furthermore, these polyethers may be in the form of a mixture.

The method of producing polyether of the general formula ( ) is not restricted to the methods given below, which are simply recited by way of example.
(a) An alcohol, represented by the general formula of R³-OH where R³ is a C₁-C₁₀ alkyl or haloalkyl group (halogen is fluorine, chlorine or bromine), is reacted with formaldehyde or its polymer, in the presence of a dehydrator such as sulfuric acid, thereby to obtain R³O(CH₂O)ₙR⁴, where R³ is the same as above, R⁴ is hydrogen, CH₃ or R³, and n is an integer of from 1 to 10.
(b) An alcohol, represented by a general formula of R³-OH where R³ is the same as above, and chloromethyl ether, represented by R⁴O(CH₂O)ₙCH₂Cl where R⁴ is the same as above, are brought into contact with a base such as caustic soda, thereby to obtain R⁴O(CH₂O)ₙCH₂OR³ where R³, R⁴ and n are the same as above.
(c) HFIPA, formaldehyde and hydrogen fluoride are reacted together in the presence of a dehydrator such as sulfuric acid, to obtain a desired polyether as a by-product in the synthesis of fluoromethyl 1,1,1,3,3,3-hexafluoroisopropyl ether.

For example, a polyether of the general formulae (1) and (2), where R¹ is hexafluoroisopropyl group, R² is methyl group, and n is 1, is obtained by the method (a) wherein HFIPA and formaldehyde and methanol as needed are reacted together, in the presence of a dehydrator such as sulfuric acid, or by the method (b) wherein HFIPA and chloromethyl methyl ether are treated with a base such as caustic soda.

According to a method of the present invention, a polyether represented by the general formula (1) is brought into contact with hydrogen fluoride, in the presence of a dehydrator as accelerant. If a polyether of the formula (2) is used HFIPA is additionally present in the reaction system. In both cases formaldehyde or a polymer thereof can be added.

Examples of the accelerant are Br ø nsted acids, such as fuming sulfuric acid, concentrated sulfuric acid, sulfuric acid, fluorosulfuric acid, phosphoric acid anhydride, phosphoric acid and trifluoromethane sulfonic acid, and Lewis acids, such as titanium tetrachloride, aluminum chloride, antimony pentachloride, aluminum trifluoride, sulfuric anhydride and antimony pentafluoride. Of these, fuming sulfuric acid, concentrated sulfuric acid, a sulfuric acid having a concentration of at least 80 wt%, fluorosulfuric acid, phosphoric acid and the like or mixtures of these are preferable.

The reaction temperature is not particularly limited, and ranges from 10 to 100 °C, preferably from 35 to 80 °C. Within this temperature range, it is possible to distill the formed fluoromethyl 1,1,1,3,3,3-hexafluoroisopropyl ether, together with the unreacted raw materials, out of the reaction system, and thus this is preferable. If it is less than 10°C, the reaction becomes impractically slow. If it exceeds 100°C, the reaction becomes too fast. With this, it becomes difficult to control the reaction, and thus this is not preferable. The reaction pressure is not particularly limited, because it has little impact on the reaction. It is generally from 1 to 10 kg/cm².

Formaldehyde may be in a form which is generally industrially available, for example, its polymers such as paraformaldehyde and trioxane, and thus in the present specification these are referred to simply as formaldehyde.

The amount of each reagent in the method of the present invention depends on the end-group type of polyether, and in the case of a mixture, on its compositional ratio. In the whole composition of the reagents, the molar ratio of the total number of moles of hexafluoroisopropyl group and/or HFIPA (hereinafter referred to as the "mole number of HFIPA") to the total number of moles of oxymethylene group and optionally fluoromethyl group or formaldehyde (hereinafter referred to as the "mole number of formaldehyde") is from 0.5 to 5, preferably from 0.7 to 3. If it is not greater than 0.5, the yield of fluoromethyl 1,1,1,3,3,3-hexafluoroisopropyl ether is lower. This is not preferable in practice. If it is not less than 5, the conversion of HFIPA reduces. This is not preferable, because the efficiency of using it decreases.

It is generally preferable that hydrogen fluoride exists stoichiometrically in excess of "the number of moles of formaldehyde". The molar ratio of that to formaldehyde is preferably from 1 to 50, more preferably from 3 to 30. If it is not greater than 1, the reaction becomes slow. This is not desired, because the yield is reduced. Even if it is not less than 50, this does not cause problems in terms of the reaction. This is not, however, particularly advantageous, because it is accompanied by an increase of the amount of the unreacted hydrogen fluoride to be removed by distillation, by an increase of the size of equipment, and the like. The molar ratio of the accelerant to formaldehyde is from 0.5 to 20, preferably from 0.7 to 5.0. If it is not greater than 0.5, the reaction rate reduces. Not less than 20 will do, but is not prefered for economic reasons.

According to the method of the present invention, there is no particular restriction on the order of the addition of each reagent. For example, there is a method in which polyether and if used HFIPA are gradually added to a mixture prepared by previously mixing the accelerant, hydrogen fluoride and optionally formaldehyde and maintained at a certain predetermined temperature. In another method the accelerant, hydrogen fluoride and polyether and optionally formaldehyde and/or HFIPA are previously mixed together at a temperature not higherthan 10°C, followed by a gradual increase of the temperature to a certain predetermined temperature. In either case, the reaction product is allowed to flow out to the outside, while relatively high boiling point components are refluxed to the reactor from the generated gas, using a condenser. The gas component, which flows out, is condensed, and is then subjected, if it is accompanied by acid, to the steps of neutralization, washing with water, drying and the like, followed by distillation, to obtain the desired fluoromethyl 1,1,1,3,3,3-hexafluoroisopropyl ether.

According to the method of the present invention, it is possible to efficiently produce fluoromethyl 1,1,1,3,3,3-hexafluoroisopropyl ether from polyether. Furthermore, it is possible to produce fluoromethyl 1,1,1,3,3,3-hexafluoroisopropyl ether from fluorinated polyethers which have been discarded in the past as by-products in the production of fluoromethyl 1,1,1,3,3,3-hexafluoroisopropyl ether. This has the substantial benefit that the production cost can be reduced substantially.

In the following, there are shown examples of embodiments of the present invention, to which the scope of the present invention is not limited. The gas chromatographic analysis was conducted in accordance with the following conditions, and % represents area %. The recovery is a reference value obtained by assuming area % = weight %.
Gas Chromatograph: Hewlett Packard HP-5890 seriesll
Column: Halomatics-624 (30mx0.32mmIDx3 *µ* m)
Column Temperature: 40°C(10 minutes maintenance) - 200°C (temperature increase rate 10°C /min)
Injection Port Temperature: 200°C
Carrier Gas: He 40kPa
Sample: 0.5 µl
Split Ratio: 1/80
Detector: FID 200°C
Integrator: Hewlett Packard HP-3396 seriesll

### [Reference Example 1] Synthesis of (CF₃)₂CHOCH₂OCH(CF₃)₂

A 300 ml reactor, which had been cooled down in dry ice/acetone bath to -20°C, was charged with 111.3 g of fuming sulfuric acid, 9.3 g of paraformaldehyde and 52.8 g of HFIPA, followed by stirring for 20 minutes. After stopping stirring, it was cooled down to room temperature while standing still. The contents were separated into two layers. The organic matter of the upper layer was taken out and then washed with 50 ml of 5% sodium hydrogencarbonate aqueous solution. With this, it was separated into two layers to obtain an organic matter.

The obtained organic matter was distilled under reduced pressure, and a distillate was recovered as a main distillate under a degree of vacuum of from 94,6 to 97,3 mbar (71 to 73 mmHg) and a distillation temperature of 55°C. With this, 21.0 g of (CF₃)₂CHOCH₂OCH(CF₃)₂ (a purity of 99.9%) was obtained. The molecular structure was determined by GC-MASS, ¹H-NMR and ¹⁹F-NMR spectrums.

(CF₃)₂CHOCH₂OCH(CF₃)₂

| Mass Spectrum | |
|---|---|
| M⁺ | 348 |
| (CF₃)₂CHOCH₂ | 181 |
| (CF₃)₂CH | 151 |
| CF₃ | 69 |

| NMR(TMS, CFCl₃ basis) | |
|---|---|
| CH₂ | δ 5.2ppm(singlet 2H) |
| CH | δ 4.5ppm(multiplet 2H) |
| CF₃ | -73.8ppm (J_{H-F} 8.3Hz doublet) |

### [Reference Example 2] Synthesis of (CF₃)₂CHO(CH₂O)₂CH(CF₃)₂

A 500 ml four-neck flask was charged with 44.5 g of paraformaldehyde, and then 120 ml of 98% sulfuric acid was added thereto with stirring under cooling with ice. While it was maintained at a temperature of up to 5°C, 127.5 g of HFIPA was added thereto. Under this condition, the reaction was continued for 1 hr. Then, a crystal, which had deposited during the reaction, was separated by filtration using a glass filter. The obtained crystal was washed with 200 ml of water, then dissolved in 1.2L of methylene chloride, and then dried with 50 g of anhydrous magnesium sulfate. After drying, methylene chloride was distilled off, to obtain 126.9 g of (CF₃)₂CHO(CH₂O)₂CH(CF₃)₂ (a purity of 99.6%). The melting point was measured, and it was 54.3°C. The molecular structure was determined by GC-MASS, ¹H-NMR and ¹⁹F-NMR spectrums.

(CF₃)₂CHO(CH₂O)₂CH(CF₃)₂

| Mass Spectrum | |
|---|---|
| M⁺ | 378 |
| (CF₃)₂CH(OCH₂)₂ | 211 |
| (CF₃)₂CHOCH₂ | 181 |
| CF₃ | 69 |

| NMR(TMS, CFCl₃ basis) | |
|---|---|
| CH₂ | δ 5.0ppm(singlet 4H) |
| CH | δ 4.4ppm(J_{H-F} 5.9Hz septet 1H) |
| CF₃ | -74.5ppm (J_{H-F} 6.0Hz doublet 3F) |

### [Reference Example 3] Synthesis of (CF₃)₂CHOCH₂OCH₃

A 500 ml four-neck flask was charged with 293 g of 15%-NaOH aqueous solution, followed by stirring. Then, 168 g of HFIPA was added thereto, followed by cooling to 10°C. 120.8 g of chloromethyl methyl ether was added thereto over a period of about 1 hr, while the temperature was maintained within a range of from 10 to 12°C. Then, stirring was continued for 30 minutes. From the two separated layers, the organic layer was removed and then washed with 200 ml of water, thereby to obtain 37.2 g of an organic matter which is (CF₃)₂CHOCH₂OCH₃ of a purity of 97.5%. This was distilled, and a main distillate was recovered at a distillation temperature of from 76 to 77°C. With this, 27.5 g of (CF₃)₂CHOCH₂OCH₃ (a purity of 99.2%) was obtained. The molecular structure was determined by GC-MASS, ¹H-NMR and ¹⁹F-NMR spectrums.

(CF₃)₂CHOCH₂OCH₃

| Mass Spectrum | |
|---|---|
| M⁺-1 | 211 |
| (CF₃)₂CHOCH₂ | 181 |
| CF₃ | 69 |
| CH₃OCH₂ | 45 |

| NMR(TMS, CFCl₃ basis) | |
|---|---|
| CH₃ | δ 3.4ppm(singlet 3H) |
| CH₂ | δ 4.8ppm(singlet 2H) |
| CH | δ 4.4ppm(multiplet 1H) |
| CF₃ | -74.5ppm (J_{H-F} 5.8Hz doublet 3F) |

### [Reference Example 4]

A 5L stainless steel reactor was charged with 500 ml of 98% sulfuric acid, 1,000 g of hydrogen fluoride and 300 g of paraformaldehyde. This reaction mixture was heated to 65°C, with stirring. Then, 1,680 g of HFIPA was added to it dropwise over a period of 2 hrs. Steam which had been generated by the reaction was collected by a water-containing trap. Then, from the two separated layers, the organic layer was removed and washed, thereby to obtain 1,410 g of an organic matter. From this organic matter, fluoromethyl 1,1,1,3,3,3-hexafluoroisopropyl ether was removed by distillation. 160 g of polyethers which are by-products were obtained as residue.

This residue was analyzed by gas chromatography and MASS, and resulted in the following finding:

| | | |
|---|---|---|
| (CF₃)₂CHO(CH₂O)ₐCH(CF₃)₂ | (a = 1-7, main component a = 1,2) | 55.9% |
| (CF₃)₂CHO(CH₂O)_{b}CH₂F | (b = 1-6, main component b = 1,2) | 31.9% |
| (CF₃)₂CHO(CH₂O)_{c}CH₃ | (c = 1-4, main component c = 1) | 1.6% |

### [EXAMPLE 1]

A1 L stainless steel reactor was charged with 75 g of 98% sulfuric acid, 196 g of hydrogen fluoride, and 124 g of polyethers obtained in the Reference Example 4, followed by gradual heating to 65°C by spending 4 hr. Steam, which had been generated by the reaction, was collected by a water-containing trap. Then, the obtained organic layer was washed with water, to obtain 126 g of an organic matter.

The obtained organic matter was analyzed by gas chromatography, and it was found to contain 96.1% of fluoromethyl 1,1,1,3,3,3-hexafluoroisopropyl ether. By distillation of this organic matter, 107 g of fluoromethyl 1,1,1,3,3,3-hexafluoroisopropyl ether (a purity of 99.9%) was obtained.

### [EXAMPLE 2]

A 200 ml stainless steel reactor was charged with 34.8 g of (CF₃)₂CHOCH₂OCH(CF₃)₂ obtained in Reference Example 1, 3 g of paraformaldehyde, 30 g of hydrogen fluoride, and 25 g of fuming sulfuric acid, followed by gradual heating to 55°C over a period of 2 hrs. Steam, which had been generated by the reaction, was collected by a water-containing trap and the organic layer obtained was washed with water, to obtain 35.3 g of an organic matter. This organic matter was analyzed by gas chromatography and was found to contain 95.3% of fluoromethyl 1,1,1,3,3,3-hexafluoroisopropyl ether (a yield of 84.1%).

### [EXAMPLE 3]

A 200 ml stainless steel reactor was charged with 37.8 g of (CF₃)₂CHO(CH₂O)₂CH(CF₃)₂ obtained in Reference Example 2, 30 g of hydrogen fluoride, and 25 g of fuming sulfuric acid, followed by gradual heating to 55°C over a period of 2 hrs. Steam, which had been generated by the reaction, was collected by a water-containing trap and the organic layer obtained was washed with water, to obtain 37.0 g of an organic matter. This organic matter was analyzed by gas chromatography, and was found to contain 94.6% of fluoromethyl 1,1,1,3,3,3-hexafluoroisopropyl ether (a yield of 87.5%).

### [EXAMPLE 4]

A 200 ml stainless steel reactor was charged with 21.2 g of (CF₃)₂CHOCH₂OCH₃ obtained in Reference Example 3, 60 g of hydrogen fluoride, and 25 g of fuming sulfuric acid, followed by gradual heating to 50°C over a period of 4 hrs. Steam, which had been generated by the reaction, was collected by a water-containing trap and the organic layer obtained was washed with water, to obtain 18.3 g of an organic matter. This organic matter was analyzed by gas chromatography and was found to contain 89.0% of fluoromethyl 1,1,1,3,3,3-hexafluoroisopropyl ether (a yield of 81.4%).

### [EXAMPLE 5]

A 1L stainless steel reactor was charged with 76 g of dimethoxymethane (CH₃OCH₂OCH₃), 120 g of hydrogen fluoride, 40 g of 98% sulfuric acid and 168 g of HFIPA, followed by gradual heating to 50°C over a period of 6 hrs. Steam, which had been generated by the reaction, was collected by a water-containing trap, and to the aqueous solution so obtained calcium chloride was added to form two layers. Then, the organic layer obtained by separation was washed with water, thereby to obtain 218.5 g of an organic matter. This organic matter was analyzed by gas chromatography, and thus it was found to contain 45.7% of fluoromethyl 1,1,1,3,3,3-hexafluoroisopropyl ether (a yield of 49.9%).

## Claims

1. A method of producing fluoromethyl 1,1,1,3,3,3-hexafluoroisopropyl ether by bringing a polyether represented by the following general formula (1) into contact with a medium comprising hydrogen fluoride, an accelerant and optionally formaldehyde.
R¹O(CH₂O)ₙR² (1)
where R¹ and R² are independently C₁-C₁₀ alkyl or haloalkyl groups, where halogen is fluorine, chlorine or bromine, n is an integer of from 1 to 10, and at least one of R¹ and R² is the (CF₃)₂CH-group, said polyether being a reactant for producing fluoromethyl 1,1,1,3,3,3-hexafluoroisopropyl ether.

2. A method of producing fluoromethyl 1,1,1,3,3,3-hexafluoroisopropyl ether by bringing a polyether represented by the following general formula (2) and 1,1,1,3,3,3-hexafluoroisopropyl alcohol into contact with a medium comprising hydrogen fluoride, an accelerant and optionally formaldehyde,
R¹O(CH₂O)ₙR² (2)
where R¹ and R² are independently hydrogen, C₁-C₁₀ alkyl or haloalkyl groups, where halogen is fluorine, chlorine or bromine, n is an integer of from 1 to 10, and both of R¹ and R² are not hydrogen at the same time, said polyether being a reactant for producing fluoromethyl 1,1,1,3,3,3-hexafuoroisopropyl ether.

3. A method of producing fluoromethyl 1,1,1,3,3,3-hexafluoroisopropyl ether according to claims 1-2, which is **characterized in that** said accelerant is fuming sulfuric acid, concentrated sulfuric acid, sulfuric anhydride, a sulfuric acid having a concentration of at least 80 wt%, or a fluorosulfuric acid.

4. A method of producing fluoromethyl 1,1,1,3,3,3-hexafluoroisopropyl ether according to claims 1-3, which is **characterized in that** said contact is conducted at a temperature of from 10 to 100°C.

5. A method of producing fluoromethyl 1,1,1,3,3,3-hexafluoroisopropyl ether according to claims 1-4, which is **characterized in that** said polyether represented by the general formulae (1) and (2) is polymethyleneglycol bishexafluoroisopropyl ether (CF₃)₂CHO(CH₂O)ₐCH(CF₃)₂ where a is an integer of from 1 to 7.

6. A method of producing fluoromethyl 1,1,1,3,3,3-hexafluoroisopropyl ether according to claims 1-4, which is **characterized in that** said polyether represented by the general formulae (1) and (2) is bishexafluoroisopropoxymethane (CF₃)₂CHOCH₂OCH(CF₃)₂.

7. A method of producing fluoromethyl 1,1,1,3,3,3-hexafluoroisopropyl ether according to claims 1-4, which is **characterized in that** said polyether represented by the general formulae (1) and (2) is polymethyleneglycol fluoromethyl hexafluoroisopropyl ether (CF₃)₂CHO(CH₂O)_{b}CH₂F where b is an integer of from 1 to 6.

8. A method of producing fluoromethyl 1,1,1,3,3,3-hexafluoroisopropyl ether according to claims 1-4, which is **characterized in that** said polyether represented by the general formulae (1) and (2) is fluoromethoxymethyl hexafluoroisopropyl ether (CF₃)₂CHOCH₂OCH₂F.

9. A method of producing fluoromethyl 1,1,1,3,3,3-hexafluoroisopropyl ether according to claims 1-4, which is **characterized in that** said polyether represented by the general formulae (1) and (2) is polymethyleneglycol methylhexafluoroisopropyl ether (CF₃)₂CHO(CH₂O)_{c}CH₃ where c is an integer of from 1 to 4.

10. A method of producing fluoromethyl 1,1,1,3,3,3-hexafluoroisopropyl ether according to claims 1-4, which is **characterized in that** said polyether represented by the general formulae (1) and (2) is methoxymethyl hexafluoroisopropyl ether (CF₃)₂CHOCH₂OCH₃.

## Patentansprüche

1. Verfahren zur Herstellung von Fluormethyl-1,1,1,3,3,3-hexafluorisopropyl-ether, bei dem ein Polyether der folgenden allgemeinen Formel (1) mit einem Medium in Kontakt gebracht wird, das Fluorwasserstoff, einen Beschleuniger und wahlweise Formaldehyd enthält,
R¹O(CH₂O)ₙR² (1)
in der R¹ und R² unabhängig voneinander C₁-C₁₀ Alkyl- oder Halogenalkylgruppen sind, wobei Halogen Fluor, Chlor oder Brom ist, n eine ganze Zahl von 1 bis 10 ist und wenigstens eine von R¹ und R² die (CF₃)₂CH-Gruppe ist, wobei der Polyether ein Reaktand zur Herstellung von Fluormethyl-1,1,1,3,3,3-hexafluorisopropyl-ether ist.

2. Verfahren zur Herstellung von Fluormethyl-1,1,1,3,3,3-hexafluorisopropyl-ether, bei dem ein Polyether der folgenden allgemeinen Formel (2) und 1,1,1,3,3,3-Hexafluorisopropylalkohol mit einem Medium in Kontakt gebracht werden, das Fluorwasserstoff, einen Beschleuniger und wahlweise Formaldehyd enthält,
R¹O(CH₂O)ₙR² (2)
in der R¹ und R² unabhängig voneinander Wasserstoff, C₁-C₁₀ Alkyl- oder Halogenalkylgruppen sind, wobei Halogen Fluor, Chlor oder Brom ist, n eine ganze Zahl von 1 bis 10 ist und R¹ und R² nicht beide gleichzeitig Wasserstoff sind, wobei der Polyether ein Reaktand zur Herstellung von Fluormethyl-1,1,1,3,3,3-hexafluorisopropyl-ether ist.

3. Verfahren zur Herstellung von Fluormethyl-1,1,1,3,3,3-hexafluorisopropyl-ether nach Ansprüchen 1 und 2, das **dadurch gekennzeichnet ist, daß** der Beschleuniger rauchende Schwefelsäure, konzentrierte Schwefelsäure, Schwefelsäureanhydrid, eine Schwefelsäure mit einer Konzentration von wenigstens 80 Gew.-% oder eine Fluorschwefelsäure ist.

4. Verfahren zur Herstellung von Fluormethyl-1,1,1,3,3,3-hexafluorisopropyl-ether nach Ansprüchen 1 bis 3, das **dadurch gekennzeichnet ist, daß** die Kontaktierung bei einer Temperatur von 10 bis 100°C durchgeführt wird.

5. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Polymethylenglykol-bishexafluorisopropyl-ether (CF₃)₂CHO(CH₂O)ₐCH(CF₃)₂ ist, in dem a eine ganze Zahl von 1 bis 7 ist.

6. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Bishexafluorisopropoxymethan (CF₃)₂CHOCH₂OCH(CF₃)₂ ist.

7. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Polymethylenglykolfluormethyl-hexafluorisopropyl-ether (CF₃)₂CHO(CH₂O)_{b}CH₂F ist, in dem b eine ganze Zahl von 1 bis 6 ist.

8. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Fluormethoxymethyl-hexafluorisopropylether (CF₃)₂CHOCH₂OCH₂F ist.

9. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Polymethylenglykolmethyl-hexafluorisopropyl-ether (CF₃)₂CHO(CH₂O)_{c}CH₃ ist, in dem c eine ganze Zahl von 1 bis 4 ist.

10. Verfahren zur Herstellung von Fluormethyl-1,1,1,3,3,3-hexafluorisopropyl-ether nach Ansprüchen 1 bis 4, das **dadurch gekennzeichnet ist, daß** der Polyether der allgemeinen Formeln (1) und (2) Methoxymethyl-hexafluorisopropyl-ether (CF₃)₂CHOCH₂OCH₃ ist.

## Revendications

1. Méthode de production de fluorométhyl 1,1,1,3,3,3-hexafluoroisopropyl éther en portant un polyéther représenté par la formule générale (1) qui suit en contact avec un milieu comprenant du fluorure d'hydrogène, un accélérant et facultativement du formaldéhyde,
R¹O(CH₂O)ₙR² (1)
où R¹ et R² sont indépendamment des groupes alkyle ou haloalkyle C₁-C₁₀, où halogène est fluor, chlore ou brome, n est un entier de 1 à 10, et au moins l'un de R¹ et R² est le groupe (CF₃)₂CH, ledit polyéther étant un réactant pour produire du fluorométhyl 1,1,1,3,3,3-hexafluoro-isopropyl éther.

2. Méthode de production de fluorométhyl 1,1,1,3,3,3-hexafluoroisopropyl éther en portant un polyéther représenté par la formule générale (2) qui suit et de l'alcool 1,1,1,3,3,3-hexafluoroisopropylique en contact avec un milieu comprenant du fluorure d'hydrogène, un accélérant et facultativement du formaldéhyde,
R¹O(CH₂O)ₙR² (2)
où R¹ et R² sont indépendamment hydrogène, des groupes alkyle ou haloalkyle C₁-C₁₀, où halogène est fluor, chlore ou brome, n est un entier de 1 à 10, et R¹ et R² ne sont pas tous deux hydrogène en même temps, ledit polyéther étant un réactant pour produire du fluorométhyl 1,1,1,3,3,3-hexafluoro-isopropyl éther.

3. Méthode de production de fluorométhyl 1,1,1,3,3,3-hexafluoroisopropyl éther selon les revendications 1-2, qui est **caractérisée en ce que** ledit accélérant est de l'acide sulfurique fumant, de l'acide sulfurique concentré, de l'anhydride sulfurique, un acide sulfurique ayant une concentration d'au moins 80% en poids, ou un acide fluorosulfurique.

4. Méthode de production de fluorométhyl 1,1,1,3,3,3-hexafluoroisopropyl éther selon les revendications 1-3, qui est **caractérisée en ce que** ledit contact est entrepris à une température de 10 à 100°C.

5. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est du polyméthylèneglycol bishexafluoroisopropyl éther (CF₃)₂CHO(CH₂O)ₐCH(CF₃)₂ où a est un entier de 1 à 7.

6. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est le bishexafluoroisopropoxyméthane (CF₃)₂CHOCH₂OCH(CF₃)₂.

7. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est le polyméthylèneglycol fluorométhyl hexafluoroisopropyl éther (CF₃)₂CHO(CH₂O)_{b}CH₂F où b est un entier de 1 à 6.

8. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est le fluorométhoxyméthyl hexafluoroisopropyl éther (CF₃)₂CHOCH₂OCH₂F.

9. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est le polyméthylèneglycol méthyl-hexafluoroisopropyl éther (CF₃)₂CHO(CH₂O)_{c}CH₃ où c est un entier de 1 à 4.

10. Méthode de production de fluorométhyl 1,1,1,3,3,3-hexafluoroisopropyl éther selon les revendications 1-4, qui est **caractérisée en ce que** ledit polyéther représenté par les formules générales (1) et (2) est le méthoxyméthyl hexafluoroisopropyl éther (CF₃)₂CHOCH₂OCH₃.
